# EUROPEAN PATENT SPECIFICATION

(11) **EP 3 503 811 B1**
(45) Date of publication and mention of the grant of the patent: **20.12.2023**
(21) Application number: 17751534.3
(22) Date of filing: 28.07.2017
(51) Int. Cl.: A61B 17/00, A61F 2/24, A61B 17/12, A61B 17/22, A61B 90/00

(54) **ADJUSTABLE GUIDEWIRE RECEIVING MEMBER**
EINSTELLBARES FÜHRUNGSDRAHTAUFNAHMEELEMENT
ÉLÉMENT DE RÉCEPTION DE FIL-GUIDE RÉGLABLE

(30) Priority: 29.08.2016 US 201662380799 P; 20.12.2016 US 201662436918 P; 20.12.2016 US 201662436985 P; 23.02.2017 US 201762462776 P; 27.07.2017 US 201715662066
(43) Date of publication of application: 03.07.2019
(73) Proprietor: Cephea Valve Technologies, Inc., Santa Clara, CA 95054 (US)
(72) Inventor: VON OEPEN, Randolf, Aptos, CA 95003 (US); MCNIVEN, Sean A., Menlo Park, CA 94025 (US); VALENCIA, Francisco, East Palo Alto, CA 94303 (US)
(74) Representative: Boult Wade Tennant LLP
(86) International application number: PCT/US2017/044475
(87) International publication number: WO 2018/044449

(56) References cited:
- WO-A1-2015/191938
- WO-A2-01/51114
- US-A- 5 415 664
- US-A1- 2001 047 150
- US-A1- 2007 203 561
- US-A1- 2010 217 261
- US-A1- 2011 307 049
- US-A1- 2013 103 001
- US-A1- 2014 148 889
- US-B1- 6 228 110

## Description

### BACKGROUND OF THE DISCLOSURE

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure. An artificial valve is guided to the heart through the patient's vasculature. For example, a catheter is inserted into the patient's vasculature and directed to the inferior vena cava. The catheter is then urged through the inferior vena cava toward the heart by applying force longitudinally to the catheter. Upon entering the heart from the inferior vena cava, the catheter enters the right atrium. The distal end of the catheter may be deflected by one or more wires positioned inside the catheter. Precise control of the distal end of the catheter allows for more reliable and faster positioning of a medical device and/or implant and other improvements in the procedure.

The devices can also be directed through the valve chordae or papillary muscles, for example, for interventional therapy to the mitral valve. When such procedures require the use of more than one instrument, each instrument would be dependent upon proper positioning in relation to the valve. Therefore, positioning or steering mechanisms need to be built into each instrument. This adds further cost, complexity, and time to the procedures.

Other procedures may include tracking a catheter and/or access sheath from a puncture in the femoral vein through the intra-atrial septum to the left atrium. This pathway may be used to access the left atrium for ablation of the atrium wall or ablation around the pulmonary veins. Such interventional therapies would require precise alignment with target areas for proper ablation placement. Additionally, alternative access routes and/or access routes to other cavities may be desired.

In US 2011/0307049 there is described a bi-directional stent delivery system that includes an inner elongate shaft, a radially expandable prosthesis disposed over the inner elongate shaft, an outer elongate shaft, and a shuttle sheath disposed over the radially expandable prosthesis. The distal portion of the inner shaft is releasably coupled to the distal portion of the shuttle sheath, and the distal portion of the outer shaft is releasably coupled the proximal portion of the shuttle sheath. Distal advancement of the inner shaft advances the shuttle sheath distally when the outer shaft is uncoupled from the shuttle sheath, thereby allowing the prosthesis to radially expand from a proximal end to a distal end. Proximal retraction of the outer shaft retracts the shuttle sheath proximally when the inner shaft is uncoupled from the shuttle sheath, thereby allowing the prosthesis to radially expand from a distal end to a proximal end thereof.

In US 5,415,664 there is described a stent, stent-graft or endolumimal graft introducer that includes a hollow tube having a proximal end and a distal end and a cup-like end cap for covering the distal end. The cap is coupled to a control member which extends through the hollow tube. The proximal ends of the tube and the control member are coupled to an actuation device for effecting relative movement of the tube and control member to move the cap relative to the distal end of the tube. The distal end of a stent or stent-graft is inserted into the cup-like cap and the actuation device is manipulated to move the cap and the distal end of the tube together, thereby gripping the distal end of the stent. The introducer is inserted into a sheath and pulls the distal end of the stent into the sheath, thereby stretching it to its reduced diameter. The sheath containing the stent and the introducer is maneuvered to the site for deployment of the stent. The introducer is held in a stationary position and the sheath is withdrawn so that a portion of the stent is released from the sheath. The introducer, stent, and sheath can then be moved as one so that the stent is precisely located before it is deployed. When the stent is in the precise location, the sheath is completely withdrawn from the stent. The actuation device is then manipulated to release the distal end of the stent from the introducer. The introducer is removed through the lumen of the stent.

In US 2007/0203561 prosthetic valves and their component parts are described, as are prosthetic valve delivery devices and methods for their use. The prosthetic valves are said to be particularly adapted for use in percutaneous aortic valve replacement procedures. The delivery devices are said to be particularly adapted for use in minimally invasive surgical procedures. The preferred delivery device includes a catheter having a deployment mechanism attached to its distal end, and a handle mechanism attached to its proximal end. A plurality of tethers are provided to selectively restrain the valve during deployment. A number of mechanisms for active deployment of partially expanded prosthetic valves are also described.

In US 6,228,110 there are described catheters which employ a variety of devices for preventing unwanted motion of the catheter tip. These methods include the use of a slidably mounted annular shim located in the tip of the catheter which may frictionally engage the guidewire element, and an annular brush with bristles. In the latter case, the extent of the frictional engagement between the tip and the guidewire is determined by the orientation of the bristles. A catheter is also described in which unwanted motion is prevented by the presence of a tension/compression cable extending from the proximal end of the catheter shaft to the distal end of the catheter shaft. In the unlocked position, the tension/compression cable has slack in it while in the engaged position, the cable is either under tension or under compression.

In WO 01/51114 there are described methods for making a loaded catheter assembly for delivering a self-expanding stent where the self-expanding stent is carried in a compressed state and the compressed stent has an inside diameter smaller than the outside diameter of the catheter distal tip. The methods utilize catheter sub-assemblies lacking already attached tips or having partially formed distal tips. A stent can be proximally and co-axially slid over the distal end of the catheter shaft and constrained by a retractable sheath disposed co-axially about the compressed stent. The catheter distal tip can be added or more fully formed after the loading of the stent. Some catheters include a preformed distal conical tip held in position by a heat-shrink film. Other catheters have an elastomeric distal tip waist for slipping over and engaging an outward projection on the catheter shaft distal region. Some catheters are adapted to engage catheter shaft distal threaded regions.

In US 2014/148889 there are described distal tips for use with delivery catheters that are configured to maintain complete engagement between the distal tip and a distal opening of a sheath component of the delivery catheter so as to prevent separation therebetween and/or to prevent fish-mouthing of a distal leading edge of the sheath component during in vivo use. Distal tips so configured realize one or more of the objectives of safer tracking of the delivery catheter through the vasculature, safe crossing of the delivery catheter through structural components of the vasculature and heart, such as through native valves, and safe removal of the delivery catheter post deployment.

In US 2001/047150 there is described a catheter for delivery of an expandable intracorporeal device and a method of using the catheter. The catheter may have an elongate shaft with a proximal section, distal section, proximal end and distal end. The distal section has a radially expandable shear barrier which is at least partially radially constrained by an outer radially constraining section. An expandable intracorporeal device, specifically an expandable endovascular graft, is disposed within an inner space within the radially expandable shear barrier. The catheter is guided to a desired site within a patient's body and the radial constraint of the outer radially constraining section is at least partially removed from the radially expandable shear barrier so as to allow the shear barrier and expandable intracorporeal device to expand and deploy. Typically, the radial constraint of the outer radially constraining section is carried out by relative axial movement between the outer radially constraining section and the radially expandable shear barrier.

### BRIEF SUMMARY OF THE DISCLOSURE

According to the present invention there is provided an intravascular device delivery system comprising the features of claim 1.

In one embodiment, the intravascular device delivery system has an elongated member with an outer sleeve and a distal cap fixed to a guidewire receiving member. The guidewire receiving member is moveable longitudinally relative to the outer sleeve to move the distal cap relative to the outer sleeve. The distal cap is moveable in a proximal direction to complimentarily mate with a distal end of the outer sleeve and moveable in a distal direction to provide clearance between the distal cap and the outer sleeve when accommodating an intravascular device.

In one embodiment, the intravascular device delivery system includes an intravascular device positioned in the outer sleeve and proximal the distal cap. The intravascular device can be a permanently implanted device or a temporarily implanted device.

In one embodiment, the intravascular device delivery system includes a handle connected to the proximal end of the guidewire receiving member. The handle includes a proximal end plate that is moveable relative to a body of the handle and that is selectively fixable relative to the body of the handle. The guidewire receiving member can be selectively fixed to the proximal end plate.

In one embodiment, the intravascular device delivery system includes a distal cap and a guidewire with one or more torque transmission features protruding in a transverse direction from a longitudinal axis of the guidewire. Those features are received in complementary receiving recesses of the distal cap.

In one embodiment, the intravascular device delivery system includes a distal cap including a distal cap end portion and a distal cap ring. The distal cap end portion being received by the distal cap ring coupled to the guidewire receiving member.

In one embodiment, the intravascular device delivery system includes a distal cap with a distal cap end portion and a distal cap proximal portion. The distal cap end portion mechanically engages with a locking ring selectively mounted to the guidewire receiving member. The locking ring slidable cooperates with the guidewire receiving member and aids with mounting the distal cap to the guidewire receiving member.

This summary is provided to introduce a selection of concepts that are further described below in the detailed description. This summary is not intended to be used in limiting the scope of the claimed subject matter.

Additional features will be set forth in the description which follows. These and other features will become more fully apparent from the following description and appended claims, or may be learned by the practice of such exemplary arrangements as set forth hereinafter.

### BRIEF DESCRIPTION OF THE DRAWINGS

In order to describe the manner in which the above-recited and other features of the disclosure can be obtained, a more particular description will be rendered by reference to specific examples thereof which are illustrated in the appended drawings. For better understanding, the like elements have been designated by like reference numbers throughout the various accompanying figures. While some of the drawings may be schematic or exaggerated representations of concepts, at least some of the drawings may be drawn to scale. Understanding that the drawings depict some exemplary arrangements, the arrangements will be described and explained with additional specificity and detail through the use of the accompanying drawings in which:
FIG. 1 is a schematic representation of an intravascular device delivery system with an atraumatic cap;
FIG. 2A is a detail perspective view of one example of a distal end of the intravascular device delivery system of FIG. 1;
FIG. 2B is a cross-sectional view of a portion of a guidewire receiving member of the intravascular delivery system of FIG. 1;
FIG. 3 is a detail cross-sectional side view of the distal end of the intravascular device delivery system of FIG. 1;
FIG. 4 is a cross-sectional view of one example of the elongated member of the intravascular device delivery system of FIG. 1;
FIG. 5 is a detailed view of one example of the handle of the intravascular device delivery system of FIG. 1;
FIG. 6 is a partial plan view of one example of a locking mechanism associated with the handle of the intravascular device delivery system of FIG. 1;
FIG. 7 is a cross-sectional view of another example of a distal end of an intravascular device delivery system;
FIG. 8A is a side view of one example of a guidewire of an intravascular device delivery system;
FIG. 8B is an end view of a distal cap of an intravascular delivery system;
FIG. 9 is a side view of another example of a distal end of an intravascular device delivery system;
FIG. 10 is an end view of the another example of the distal end of an intravascular device delivery system;
FIG. 11 is a side view of another example of a distal end of an intravascular device delivery system;
FIG. 12 is a side view of the distal end of the intravascular device delivery system of FIG. 11;
FIG. 13 is a cross-sectional view of a fixture associated with the intravascular device delivery system;
FIG. 14 is a side view of another example of a distal end of an intravascular device delivery system;
FIG. 15 is a side view of another example of a distal end of an intravascular device delivery system;
FIG. 16 is a side view of a distal end of an intravascular device delivery system in accordance with the invention;
FIG. 17 is a side cross-sectional view of another example of a distal end of an intravascular device delivery system;
FIG. 18 is a side cross-sectional view of another example of a distal end of an intravascular device delivery system; and
FIG. 19 is a side view of a portion of a distal end of another example of an intravascular device delivery system.

### DETAILED DESCRIPTION

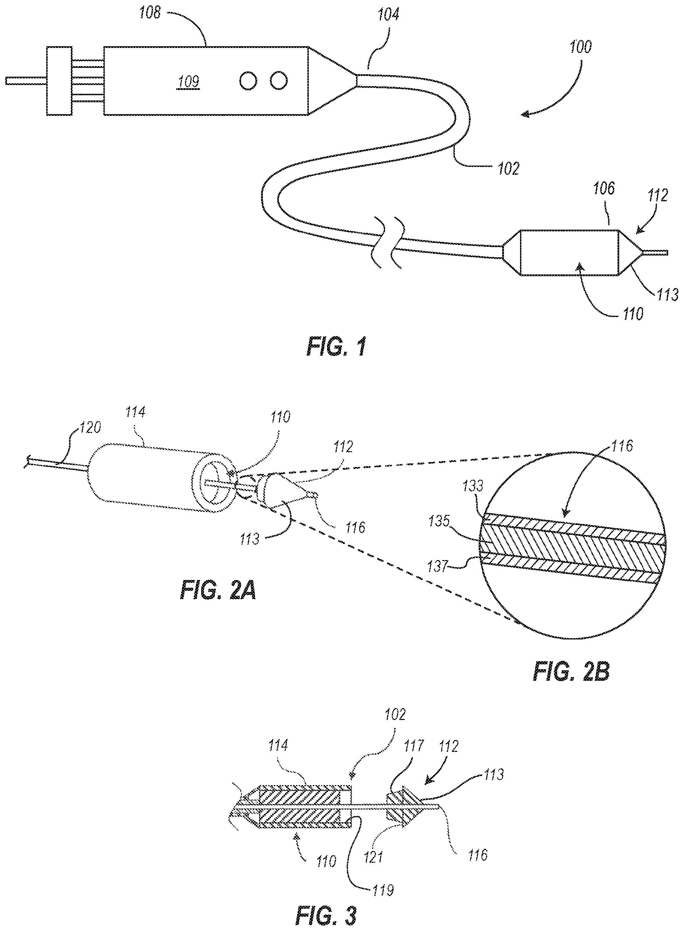

The present disclosure generally relates to manufacturing and using intravascular device delivery systems or other steerable intravascular systems. An intravascular device delivery system allows a medical professional to deliver an intravascular or other medical device to a target location in a patient's body. While the present disclosure will describe intravascular device delivery systems and applications thereof in relation to intravascular procedures in the heart, it should be understood that the devices, systems, and methods described herein may be applicable to other bodily lumens and/or cavities. Additionally, elements described in relation to any arrangement depicted and/or described herein may be combinable with elements described in relation to any other arrangement depicted and/or described herein. For example, and not by way of limitation to other specific combinations, any element described in relation to the arrangements depicted in FIGS. 2A, 2B, 3, 7-12, or 14-19 may be combinable with any element of the arrangements described in FIG. 1, 4, 5, and 13,.

An intravascular device delivery system, in one configuration, may include a flexible elongated member that has a distal end and a proximal end. A handle may be connected to a proximal end of the elongated member to allow a user, such as a medical professional and/or clinician, to control one or more movements of the elongated member. An intravascular device may be positioned at and/or connected to the distal end of the elongated member.

The distal end of the elongated member may include a distal cap that is atraumatic and includes at least a part that is soft to prevent damage to bodily tissue. The distal cap is positioned at the distal end of the elongated member to selectively limit movement of an intravascular device in the distal direction. The distal cap is movable relative to an outer sleeve of the elongated member in a longitudinal direction to selectively allow the intravascular device to be deployed and/or delivered at a target location in the patient's body. For example, the distal cap is operably connected to the handle of the intravascular device delivery system to control the longitudinal position of the distal cap relative to the outer sleeve.

The handle may include one or more controls (e.g., a knob, a button, a lever, or other controls) that may move at least one part of the intravascular device delivery system relative to another. For example, the handle may include one or more controls for moving at least one element of the elongated member relative to another element of the elongated member. The handle may move an inner element relative to an outer element of the elongated member in a proximal direction, in a distal direction, in a rotational direction, or combinations thereof.

FIG. 1 illustrates a schematic representation of an intravascular device delivery system 100. The system 100 may include an elongated member 102 having a proximal end 104 and a distal end 106. One or more handles 108 may be connected to the proximal end 104 of the elongated member 102. An intravascular device 110 may be positioned at and/or connected to the distal end 106. A distal cap 112 is positioned at the distal end 106 and configured to limit the movement of the intravascular device 110.

The elongated member 102 may be flexible, allowing the elongated member 102 to traverse a patient's tortuous vasculature or other anatomy. In some procedures, the elongated member 102 may deliver the intravascular device 110 to a target location in the patient's body, such as delivering a filter, scaffold, stent, body tissue repair device, heart valve, or other implantable devices. In other procedures, the system 100 and elongated member 102 may be provided without an intravascular device 110 at the distal end 106 such that the system may recapture, reposition, or otherwise move an intravascular device previously positioned in the patient's body or the elongate member 102 or the system 100 can be used as part of a diagnostic or other therapeutic device.

The elongated member 102 of the system 100 may include one or more elements therein. An element of the elongated member 102 may include a catheter, a guidewire, a sheath, a drive cable, other tubular and/or solid element, or combinations thereof. In some arrangements, an element of the elongated member 102 may extend an entire length of the elongated member 102 from a proximal end 104 to a distal end 106 of the elongated member 102. In other arrangements, an element of the elongated member 102 may have a length less than the entire length of the elongated member 102. For example, an element may provide support to the elongated member 102 from the proximal end 104 toward the distal end 106 without continuing the entire length to the distal end 106.

FIG. 2A is a perspective view of the distal end 106 of the elongated member 102 of FIG. 1. The distal cap 112 is displaced in a distal direction in FIG. 2 and apart from an outer sleeve 114 of the elongated member 102. The intravascular device 110 is positioned radially within the outer sleeve 114. The distal cap 112 is connected to a guidewire lumen, a guidewire receiving member, or tubular member 116 having a guidewire lumen that extends proximally from the distal cap 112 through the elongated member 102 to the handle (e.g., handle 108 shown in FIG. 1). The guidewire receiving member 116 can include an inner liner 133, a braid 135, and a cover 137 extending over the braid 135, as illustrated in FIG. 2B. FIG. 2B illustrates one portion of a wall of the guidewire receiving member 116 rather than an entire cross-section of both walls. The inner liner 133 can be polytetrafluoroethylene (PTFE) or other polymer liner, the braid 135 can be fabricated from metal, polymer, ceramic, or other combinations thereof, and the cover 137 can be a polyamide, polyimide, or other polymeric cover.

The distal cap 112 is longitudinally and/or rotationally fixed to the guidewire receiving member 116 such that moving the guidewire receiving member 116 in a longitudinal direction moves the distal cap 112 in the longitudinal direction. The distal cap 116 is tapered in the distal direction to provide an atraumatic surface that prevents or limits damage to the tortuous anatomy of a patient. In addition, the distal cap 112 can include or be formed, at least in part, of a polymeric material that is sufficiently soft so as not to damage bodily tissue. For instance, the distal cap 116 can be formed, at least in part, of Polyether block amide (PEBAX), Polyurethane, Silicone, or other materials.

In the illustrated configuration of FIG. 2A, the tapered distal end 113 also allows the distal end 106 of the elongated member 102 to traverse the intra-atrial septum in the heart, for instance. For example, a guidewire may be positioned within the guidewire lumen of the guidewire receiving member 116 and the guidewire may puncture the intra-atrial septum. The tapered distal end 113 of the distal cap 112 expands the puncture in the septum to the width of the distal end 106 of the elongated member 102 to allow the distal end 106 to cross the septum.

For loading and optionally deployment of the intravascular device, the distal cap 112 is longitudinally movable in the distal direction. In some arrangements, the distal cap 112 is movable a distance up to the longitudinal length of the intravascular device 110 positioned in the outer sleeve 114. In other arrangements, the distal cap 112 is movable a distance greater than the longitudinal length of the intravascular device 110. In yet other arrangements, the distal cap 112 is movable a distance less than the longitudinal length of the intravascular device 110. For example, the intravascular device 110 may foreshorten in the longitudinal direction in a deployed or expanded state (and conversely, lengthen when radially compressed in the undeployed or collapsed state). The intravascular device 110 may be deployed by moving the intravascular device 110 longitudinally relative to the outer sleeve 114 or sheath constraining a radial expansion of the intravascular device 110 (such as with a self-expanding stent or other self-expanding intravascular device 110). In other examples, the intravascular device 110 may have one or more movable or deformable members. In still other examples, the outer sleeve 114 is moved proximally and the distal cap 112 is maintained in its distal position during deployment.

FIG. 3 illustrates a schematic detailed cross-sectional side view of the distal end 106 of the elongated member 102 of FIG. 1. The intravascular device 110 is loaded radially within the outer sleeve 114 of the elongated member 102. The distal cap 112 is longitudinally fixed relative to the guidewire receiving member 116. The longitudinal position of the distal cap 112, therefore, may be controlled by the longitudinal movement of the guidewire receiving member 116 relative to the outer sleeve 114.

The distal cap 112 may be configured to complimentarily mate with a distal end 106 of the elongated member 102 around the intravascular device 110. The distal cap 112, therefore, has the tapered distal end 113 that tapers in the distal direction. This taper is atraumatic and prevents damage to the femoral vein or other portions of the tortuous anatomy during guiding of the intravascular device delivery device. In some procedures, the distal cap 112 can be used to enlarge an opening in the intra-atrial septum during navigation of the intravascular device delivery system to the target location. Alternatively, the hole in the septum may be sufficiently large that the distal cap 112 can pass through without enlarging the hole, such as when a physician has expanded a puncture created by a guidewire or needle using a balloon.

The distal cap 112 may also have a proximal tapered end 117 to ease centering of the distal cap 112 within the elongated member 102 and optionally cooperate with a complementary tapered inner surface 119 of the outer sleeve 114. For example, the distal cap 112 may taper in the proximal direction, or the proximal tapered end 117 can taper in the proximal direction, at 5°, 10°, 15°, 20°, 25°, 30°, 35°, 40°, 45°, 50°, or any values therebetween relative to the guidewire receiving member 116. The distal cap 112 may have a shoulder 121 positioned between the tapered distal end 113 and the proximal tapered end 117. The shoulder 121 allows the distal cap 112 to form a seal against the outer sleeve 114 at the distal end 106 of the elongated member 102 when a proximal tension force is applied to the guidewire receiving member 116 and the distal cap 112 is pulled in the proximal direction. The shoulder 121 may have a diameter that substantially matches an outer diameter of the outer sleeve 114 to provide a smooth transition between the distal cap 112 and the outer sleeve 114. Alternatively, the outer diameter of the shoulder 121 may be larger or smaller than the outer diameter of the outer sleeve 114.

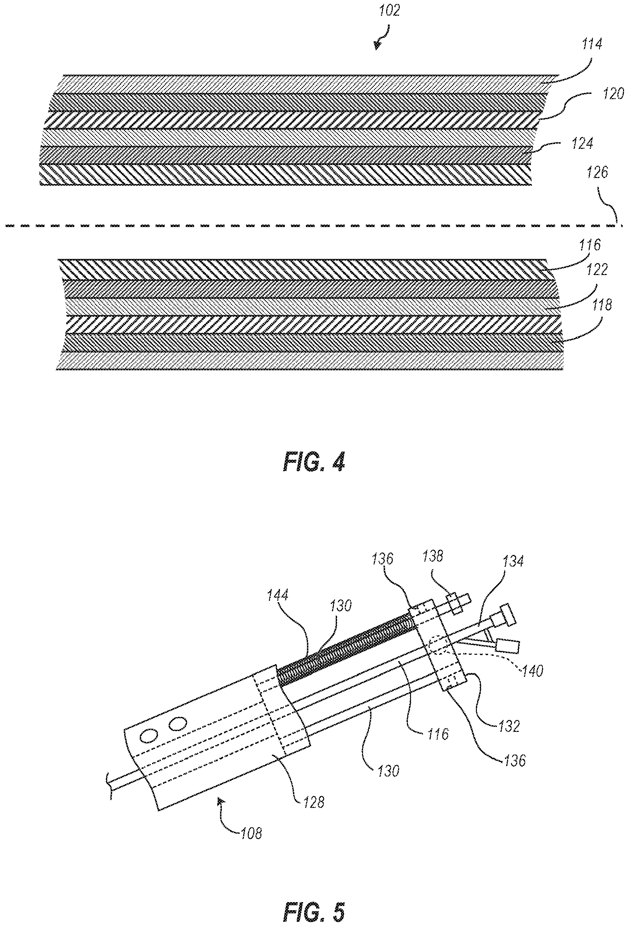

FIG. 4 is a side partial cutaway view of part of one example of the elongated member 102 of the intravascular device delivery system 100 of FIG. 1. In some arrangements, the elongated member 102 may include an outer sleeve 114, a steerable guide catheter 118, a steerable catheter 120, a compression coil 122, and an inner catheter 124. While FIG. 4 illustrates the elements of the elongated member 102 being in contact, the steerable guide catheter 118, the steerable catheter 120, the compression coil 122, and the inner catheter 124 can be separated from each other with gaps or spaces. This aids with longitudinal and rotational movement between the outer sleeve 114, the steerable guide catheter 118, the steerable catheter 120, the compression coil 122, and the inner catheter 124 based upon the particular configuration of the elongated member 102.

The guidewire receiving member 116 is positioned radially within the inner catheter 124 and parallel to a longitudinal axis 126 of the elongated member 102. While the side partial cutaway view includes the outer sleeve 114 overlapping many of the other elements of the elongated member 102, it will be understood, as illustrated in

FIG. 3, that distal ends of the steerable guide catheter 118, the steerable catheter 120, the compression coil 122, and the inner catheter 124 may terminate proximal the outer sleeve 114, so that the outer sleeve 114 extends over the intravascular device 110 (FIG. 1), or that the outer sleeve 114 include a proximal end distal the distal ends of the steerable guide catheter 118, the steerable catheter 120, the compression coil 122, and the inner catheter 124, with the outer sleeve of FIG. 4 being another outer sleeve.

One or more of the elements of the elongated member 102 may be longitudinally moveable relative to one another. In some arrangements, at least one of the elements of the elongated member is longitudinally fixed relative to another. For example, the inner catheter 124 and compression coil 122 may be longitudinally fixed relative to one another at a distal end of the inner catheter 124 and compression coil 122.

Additionally, one or more elements of the elongated member 102 may be rotationally moveable relative to one another. In some arrangements, at least one of the elements of the elongated member is rotationally fixed relative to another. For example, the outer sleeve 114 and steerable guide catheter 118 may be rotationally fixed relative to one another at an intermediate position between the proximal end and the distal end of the outer sleeve 114.

With continued reference to FIG. 4, the guidewire receiving member 116 is longitudinally and/or rotationally moveable relative to at least the inner catheter 124 of the elongated member 102. In at least one arrangement, the guidewire receiving member 116 is removable from the elongated member 102 by moving the guidewire receiving member 116 in a proximal direction. In at least one arrangement, the guidewire receiving member 116 is removable in a distal direction. In some arrangements, a guidewire lumen locking mechanism is configured to selectively fix the guidewire lumen or guidewire receiving member longitudinally and/or rotationally.

FIG. 5 illustrates one example of the handle 108 operably connected to the elongated member 102, as described in relation to FIG. 1. The handle 108 illustrated in FIG. 5 is illustrated without a housing 109 of the handle 108, which is schematically illustrated in FIG. 1. The handle 108 includes a body 128 with one or more rails 130 connected thereto. In some arrangements, such as illustrated in FIG. 5, the rails 130 are oriented in the longitudinal direction. In other arrangements, however, the rails 130 are oriented at an angle to a longitudinal axis of one or more elements of the elongated member 102 and/or the handle 108. A proximal end plate 132 is connected to the one or more rails 130.

In some arrangements, the guidewire receiving member 116 is longitudinally and/or rotationally fixed relative to a proximal end plate 132 and the proximal end plate 132 is moveable relative to the body 128. For example, the proximal end plate 132 is configured to slide on the one or more rails 130, while the guidewire receiving member 116 moves with the proximal end plate 132. The proximal end plate 132 can also include at least one locking mechanism 136 to longitudinally fix the proximal end plate 132 relative to the one or more rails 130. For instance, set screws, complementary detents, clamps, rotational locks, levers, snap or friction-fit connections, or other locking mechanisms can be used to fix the proximal end plate 132 to the rails 130.

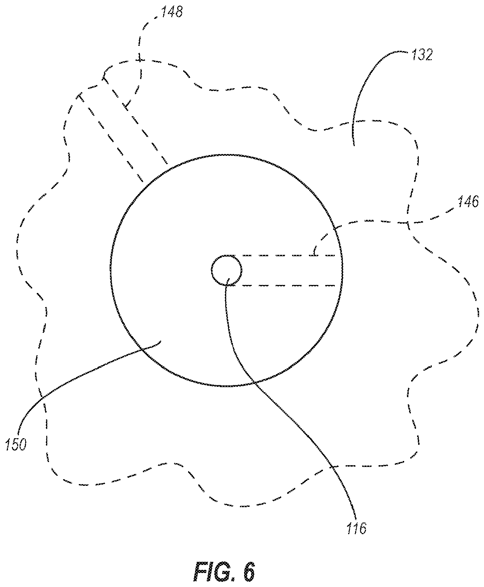

In other arrangements, the guidewire receiving member 116 is longitudinally and/or rotationally moveable relative to the proximal end plate 132 and the proximal end plate 132 is fixed relative to the body 128. For example, the proximal end plate 132 is longitudinally fixed on the one or more rails 130. In at least one arrangement, the proximal end plate 132 includes at least one locking mechanism 140 to longitudinally and/or rotationally fix the guidewire receiving member 116 relative to the proximal end plate 132. For instance, set screws, complementary detents, clamps, rotational locks, levers, snap or friction-fit connections, or other locking mechanisms can be used to fix the guidewire receiving member 116 to the proximal end plate 132. As illustrated in FIG. 6, the locking mechanism 140 can prevent longitudinal movement of the guidewire receiving member 116, while allowing rotational movement of the guidewire receiving member 116 through use of two complementary locking structures. For instance, the locking mechanism 140 includes a bearing 150 within which the guidewire receiving member 116 is received, a first set screw 146 limits movement of the guidewire receiving member 116 relative to the bearing and a second set screw 148 limits movement of the bearing relative to the proximal end plate 132.

In at least one arrangement, both the guidewire receiving member 116 is moveable relative to the proximal end plate 132 and the proximal end plate 132 is moveable relative to the body 128 of the handle 108.

With continued reference to FIGS. 1, 4, and 5, to load the intravascular device 110, the proximal end plate 132 is pushed distally to create space at the distal end of the elongated member 102 between the outer sleeve 114 and the distal cap 112. After the intravascular device 110 has been loaded, the proximal end plate 132 is moved proximally and longitudinally locked in place. This in turn positions the distal cap 112 against a distal end of the outer sleeve 114.

Because navigation through tortuous anatomy may cause one or more elements of the elongated member to displace or distort (i.e., foreshorten or stretch) in a longitudinal direction at a different rate than other elements of the elongated member, the guidewire receiving member 116 can be pre-loaded with a tension force to ensure that the distal cap 112 remains in contact with the distal end of the outer sleeve 114 during delivery of the intravascular device 110. For instance, the pre-load tension may be in the range from 4 to 44 Newtons (1 to 10 pounds of force) or less than 4 Newtons (1 pound) or greater than 44 Newtons (10 pounds). When the proximal end plate 132 is pulled proximally and a force of pre-load is applied, the proximal end plate 132 is fixed to the one or more rails 130. The force can be applied by a trained physician pulling back the proximal end plate 132. Alternatively, the pullback force is defined by a spring 144 that is placed between the proximal end plate 132 and the rails 130 and/or body 128. The force of the spring 144 will therefore define the pre-load of the guidewire receiving member 116.

To prevent excessive application of force, a distance to move the proximal end plate 132 may be limited or preset with stops 138 or other limiting structures disposed on the rails 130. Set screws, complementary detents, clamps, rotational locks, levers, snap or friction-fit connections, or other locking mechanisms can be used to fix the stops 138 to one or more rails 130. Alternatively, there might be two distinct longitudinal positions for the proximal end plate 132 relative to the body 128 to demark a space for the intravascular device 110 during loading and delivery of the intravascular device 110, while also setting a position for force application. The two distinct longitudinal positions can be defined by aligned indicia, engaging grooves or slots on the proximal end plate 132 and the rails 130 or the guidewire receiving member 116, or other mechanisms to limit movement in two different directions.

As shown in FIG. 5, the handle 108 includes a hemostasis valve 134 in communication with the guidewire receiving member 116. The hemostasis valve 134 has an open position and a closed position to selectively allow the flushing of the guidewire receiving member 116. For example, a medical profession may use the hemostasis valve 134 to control pressure differentials during and/or after navigation of the patient's anatomy using the hemostasis valve 134.

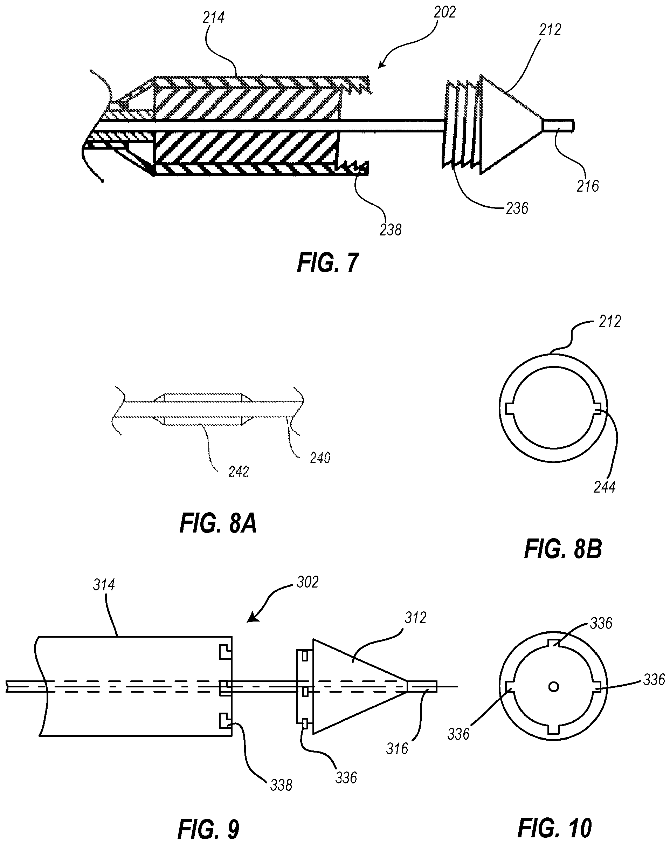

FIG. 7 illustrates another example of a distal cap 212. The discussion related to the other distal caps, guidewire receiving members, etc., described herein are also applicable to the discussion of this example of the distal cap 212, and vice versa. For instance, and not by way of limitation, the distal cap 212 is generally atraumatic and can have at least a portion, in one configuration, that is formed or fabricated of a polymeric material that will limit damage to bodily tissue.

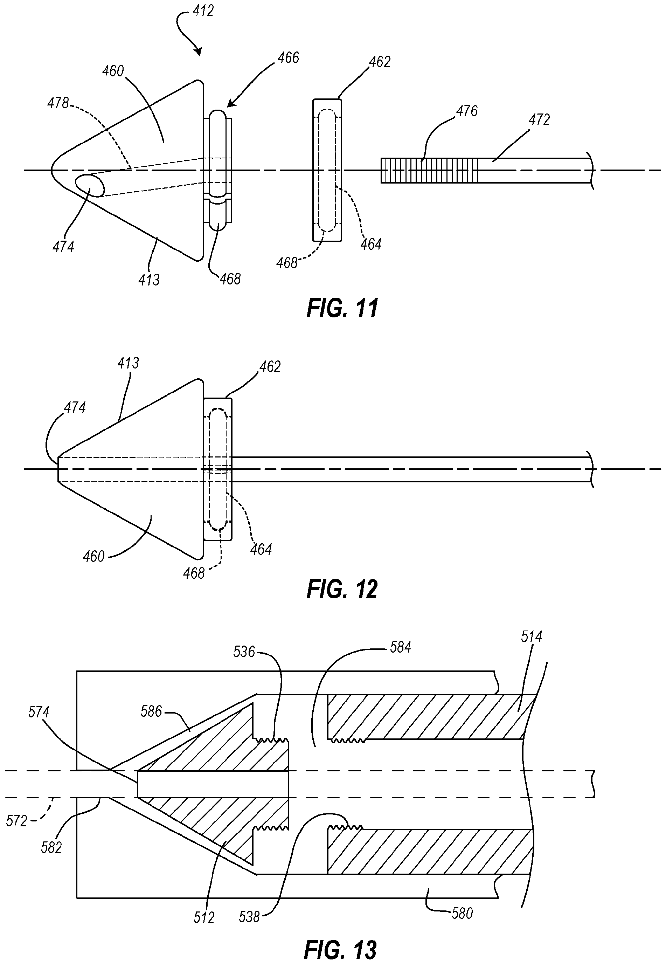

The distal cap 212 may be connected to at least a portion of the elongated member 202 to mechanically secure the distal cap 212 to the elongated member 202. In some arrangements, the distal cap 212 includes one or more mechanical connection features that engage with one or more complimentary connection features on the elongated member 202. The one or more mechanical connection features and the one or more complimentary connection features can include snaps, pins, grooves, rims, other textured surfaces, or combinations thereof. In addition, the distal cap 212 shown in FIG. 7 includes a threaded surface 236 on the distal cap 212 that can engage with a complimentarily threaded surface 238 on an inner surface of the outer sleeve 214. The threads of these threaded portions can be extremely fine. With the wall of the outer sleeve 214 being about 0.2 mm in thickness, the thread depth can be of 0.1 mm or less. Aligning the threads through use of a fixture, such as the fixture 580 of FIG. 13 that will be discussed hereinafter, limits the possibility of missalignment and possible distruction of the threads.

To engage the threaded surfaces (or other rotationally engaging connection features), torque may be transmitted to the distal cap 212 through the guidewire receiving member 216 or alternatively by a physician, clinician, health care specialist, nurse, etc. rotating the distal cap 212 onto the outer sleeve 214 after placement of the intravascular device 110 within the outer sleeve 214 and before positioning within a patient's vasculature. When applying torque through the guidewire receiving member 216, such as a polyamide guidewire receiving member 216, the guidewire receiving member 216 may have insufficient torsional strength to transmit sufficient torque to disengage the distal cap 212 from the outer sleeve 214. In some arrangements, the guidewire receiving member 216 may include one or more cables, braids, or coils to reinforce the guidewire receiving member 216. In at least one arrangement, the guidewire receiving member 216 may be a hypotube, a braided tube, or a cable tube. A cable tube is a tube formed of cables and is very flexible, yet can transmit torque and/or pushing forces. The cable tube can be comprised of 1 or more layers of cables. Helical Hollow Strand (HHS) Tube, manufactured by Fort Wayne Metals, is an example. In some arrangements, the guidewire receiving member 216 may be insufficient to disengage the distal cap 212 from the outer sleeve 214 and the guidewire itself may be used to transmit torque from the handle to the distal cap 212, such as the guidewire illustrated in FIGS. 8A and 8B.

As shown in FIG. 8A, a guidewire 240 can have one or more torque transmission features 242 protruding in a transverse direction from the guidewire 240. These torque transmission features 242 can be wings, arms, protrusions, or other structures that can mate or engage with a complementary structure in the distal cap 212. For instance, the one or more torque transmission features 242 may be received in complimentarily shaped recesses 244 in the distal cap 212, as illustrated in FIG. 8B. A procedure for using this keyed guidewire 240 and the complementarily shaped recess 244 may include loading the intravascular device 110 into the outer sleeve 214, engaging the distal cap 212 with the outer sleeve 214 to connect the distal cap 212 to the elongated member 202, inserting the keyed guidewire 240 into the vasculature of the patient, and inserting the delivery system over the guidewire 240. After the intravascular device 110 has been positioned at the target location, pulling the keyed guidewire 240 proximally to engage the one or more torque transmission features 242 with the complementary features 244 of the distal cap 212, torqueing the guidewire 240 to unscrew the distal cap 212, advancing the guide wire receiving member 216 to create the space to deliver the intravascular device 110, and deploying the intravascular device 110.

FIGS. 9 and 10 illustrate another example of a distal cap 312 that is similar to the previously described distal caps, including but not limited to distal caps 112 and 212. As such, the disclosure related to those distal caps, and guidewire receiving members, is also applicable to the disclosure of the distal cap 312, and vice versa. For instance, and not by way of limitation, the distal cap 312 is generally atraumatic and can have at least a portion, in one configuration, that is formed or fabricated of a polymeric material that will limit damage to bodily tissue.

The distal cap 312 may be connected to at least a portion of the elongated member 302 mechanically securing the distal cap 312 to the elongated member 302. In some arrangements, the distal cap 312 includes one or more mechanical connection features thereon that engage with one or more complimentary connection features on the elongated member 302. For example, the distal cap 312 shown in FIG. 9 includes pins 336 on the distal cap 312 that may engage with complimentarily slots 338 of the outer sleeve 314.

To engage the pins 336 and slots 338 (or other rotationally engaging connection features), torque may be transmitted to the distal cap 312 through the guidewire receiving member 316 or alternatively by a physician, clinician, health care specialist, nurse, etc. rotating the distal cap 312 onto the outer sleeve 314 after placement of the intravascular device 110 within the outer sleeve 314 and before positioning within a patient's vasculature. The distal cap 312 is slid into engagement with the outer sleeve 314 and the torque may be transmitted to the distal cap 312 to capture the pins 336 within the slot 338 to mechanically connect or couple the distal cap 312 to the outer sleeve 314. While the slots 338 are generally illustrated as having an L-shape it will be understood that other shapes and orientations are appropriate to facilitate mechanical connection between the pins 336 of the distal cap 312 and the outer sleeve 314. Further, the pins 336 can include tapered, chamfered, curved, or other surface structures to aid with engagement between the distal cap and the outer sleeve.

A polyamide guidewire receiving member 316 may have insufficient torsional strength to transmit sufficient torque to disengage the distal cap 312 from the outer sleeve 314. In some arrangements, therefore, the guidewire receiving member 316 may include one or more cables or coils to reinforce the guidewire receiving member 316. In at least one arrangement, the guidewire receiving member 316 may be a hypotube, a braided tube, or a cable tube. In some arrangements, the guidewire receiving member 316 may be insufficient to disengage the distal cap 312 from the outer sleeve 314 and the guidewire receiving member 316 itself may be used to transmit torque from the handle to the distal cap 312.

FIGS. 11 and 12 illustrate another example of a distal cap 412. The discussion related to the other distal caps, guidewire receiving members, etc., described herein are also applicable to the discussion of this example of the distal cap 412, and vice versa. For instance, and not by way of limitation, the distal cap 412 is generally atraumatic and can have at least a portion, in one configuration, that is formed or fabricated of a polymeric material that will limit damage to bodily tissue.

The distal cap 412 has a two-part form, with a distal cap end portion 460 and a distal cap ring 462, the distal cap end portion 460 being received in an opening 464 of the distal cap ring 462. A proximal end 466 of the distal cap end portion 460 and the opening 462 of the distal cap ring 462 have complementary engagement features 468, such as detents, radial recesses and protrusions, or other mechanical engagement features that allow the distal cap end portion 460 and the distal cap ring 462 to couple together, for example, through a snap-fit or friction fit engagement. An adhesive or glue can also be used, either alone or in combination with the complementary engagement features 468, to secure the distal cap end portion 460 to the distal cap ring 462. In one configuration, the engagement feature of the distal cap end portion 460 includes slots or slits 470 that aid the proximal end 466 to resiliently deform to be received in the opening 462.

The distal cap ring 462 is a metallic ring that is mounted to the guidewire receiving member 472 such as through welding, laser welding, adhesive bonding, engagement of complementary structures, and combinations of the same. The distal cap ring 462 provides a secure attachment of the distal cap end portion 460 to the guidewire receiving member 472. Although reference is made to the distal cap ring 462 being metallic, other materials are also possible, such as polymers or other materials.

The guidewire receiving member 472 extends through a channel 478 in the distal cap end portion 460 to terminate at an exit hole 474 disposed either on a side surface of the tapered distal end 413 or at or near an apex of the tapered distal end 413. The hypotube forming the guidewire receiving member 416 includes a cut pattern 476 to provide flexibility and support to the distal cap 412, and more specifically to the distal cap end portion 460 when it is formed from a soft polymer. The cut pattern 476, such as a laser cut pattern, allows the distal cap 412 to deflect between about 20° to about 30°. The angular deflection can be greater or lesser than about 20° to about 30°. For instance, the angular deflection can be about 3° to about 45°, from 3° to about 30°, from 30° to about 45°, or other angular deflections.

The inclusion of a hypotube provides flexibility to reduce the possibility of tissue damage when the distal cap 412 is moved relative to the outer sleeve 414, reduces the possibility that the guidewire will cut or damage the distal cap 412 as a result of the forces used to position the distal cap 412 to deploy the intravascular device 110, and reduces the possibility that the guidewire will kink distally of the distal cap 412 during sharp bends.

As mentioned above, the distal cap can be positioned on a distal end of the outer sleeve of the intravascular device. Described herein is a method and device to aid the alignment of the distal cap with the outer sleeve and to facilitate the attachment of the distal cap to the outer sleeve. In addition, aligning the distal cap and the outer sleeve can also aid the positioning of the guidewire receiving member in the center of the intravascular device. Maintaining the guidewire receiving member near to the center or at the center of the intravascular device, for instance, decreases the likelihood of pinching or narrowing of the guidewire receiving member by struts or structures of the intravascular device.

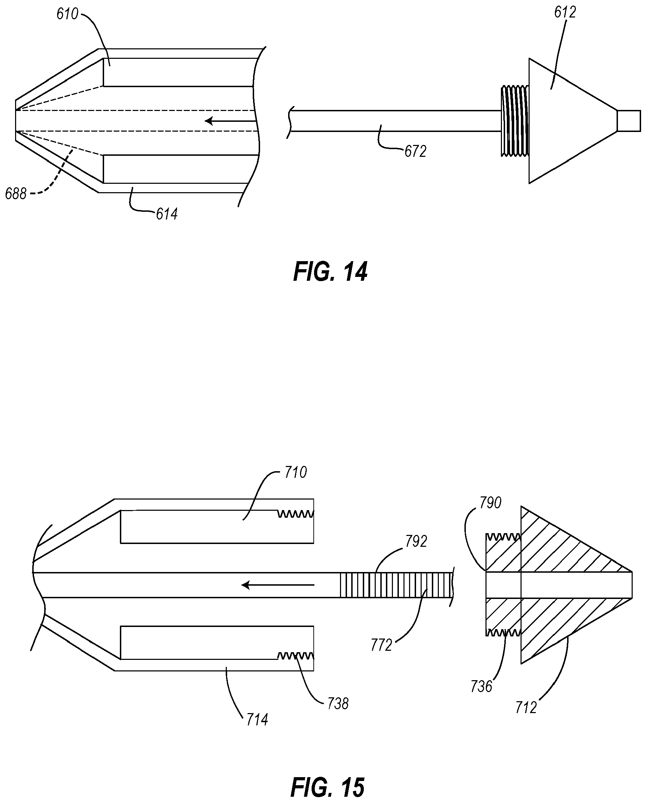

As illustrated in FIG. 13, the fixture or bushing 580, or more generally a cap alignment assembly, aids in aligning the distal cap 512, distal cap 612, or distal cap 712 of FIGS. 14 and 15, or any of the other distal caps described herein, with the outer sleeve 514, each of which can be similar to those described herein. As such, the description of any of the previously described distal caps and outer sleeves is also applicable to the description of the fixture or bushing 580.

Following loading of the intravascular device 110 into the outer sleeve 514, the fixture 580 can receive the distal cap 512 and a distal end of the outer sleeve 514. For instance, the distal cap 512 can be received within a lumen 584 so that the slanted or tapered sides of the distal cap 512 are aligned with and/or contact a tapered portion 586 and a hole 574 of the distal cap 512 aligns with a lumen or bore 582. This lumen or bore 582 can receive a portion of the guidewire receiving member 572, such as when it is used to thread the distal cap 512 to the outer sleeve 514.

Alternatively, to ensure alignment of the outer sleeve 514, following positioning of the intravascular device 110 into the outer sleeve 514, the fixture or bushing 580 can be attached to a fixture holding the outer sleeve 514. This fixture 580 can hold the distal cap 512 in line with an axis of the outer sleeve 514. Before pulling the intravascular device 110 into the outer sleeve 514, the distal cap 512 is fixed in a clamp (not shown) and a stiff stylet or a guide wire placed inside of the guidewire receiving member 572 to ensure a proper alignment. The proximal end of the guidewire receiving member 572 is not attached to the rest of the catheter when the intravacular device 110 is pulled into the outer sleeve 514. Alternatively, if the guidewire receiving member 572 is connected to a proximal end of the intravascular device delivery system, such as a catheter or catheter handle, the fixture 580 holding the cap moves simultaneously with the catheter.

As mentioned before, the outside threaded portion 536 can threadably engage with the internal threaded portion 538 of the outer sleeve 514 through rotation by a clinician, physician, technician or some other individual or alternatively can be rotated through rotation of the guidewire receiving member 572. The threads of these threaded portions can be extremely fine. With the wall of the outer sleeve 514 being about 0.2 mm in thickness, the thread depth can be 0.1 mm or less. Aligning the threads through use of the fixture 580 limits the possibility of missalignment and the possible destruction of the threads.

The fixture 580 can used for various configurations of distal cap and guidewire receiving member, such as, but not limited to, those described herein. Generally, the different configurations can include two general situations; a first, where the distal cap and the guidewire receiving member are mounted together before advancing the guidewire receiving member through the loaded or unloaded intravascular device (see FIG. 14), and a second, where the distal cap is mounted to the guidewire receiving member after it has been advanced through the loaded or unloaded intravascular device (see FIG. 15). For instance, in the first case, as illustrated in FIG. 14, when the intravascular device 610 is loaded, the distal cap 612 attached to the guidewire receiving member 672 can be reconnected to the outer sleeve 614 through a friction fit or some mechanical interlock, such as a threaded engagement or other type of mechanical interlock, as described herein. Alternatively, the combined guidewire receiving member 672 with the attached distal cap 612 can pass through a lumen or hole formed in a fixture (not shown) used to support the intravascular device 610 when it is mounted to the intravascular device 100. For instance, a center supporting member 688 can include a through-hole through which passes the combined guidewire receiving member 672 with the attached distal cap 612.

In the second case, as illustrated in FIG. 15, the distal cap 712 is selectively mounted to the guidewire receiving member 772 having the form of a cable tube. The discussions related to the other distal caps, guidewire receiving members, etc., described herein are also applicable to the discussion of this example of the distal cap 712, and vice versa. For instance, and not by way of limitation, the distal cap 712 is generally atraumatic and can have at least a portion, in one configuration, that is formed or fabricated of a polymeric material that will limit damage to bodily tissue. For instance, the distal cap 712 can include an internal threaded lumen 790 that can be threaded onto a complementary thread 792 of the guidewire receiving member 772 in one direction, while also including an outside threaded portion 736 which can threadably connect with an internal threaded portion 738 of the outer sleeve 714 in another direction opposite to the one direction. Thus, the internal threaded lumen 790 of the distal cap 712 has a reverse thread direction compared to the outside threaded portion 736. This allows attachment and detachment of the distal cap 712 from the outer sleeve 714 without the distal cap 712 detaching from the guidewire receiving member 772. As with the previous configuration, once the intravascular device 710 is loaded, the distal cap 712 is threadably engaged with the guidewire receiving member 772 and then reconnected to the outer sheath 714.

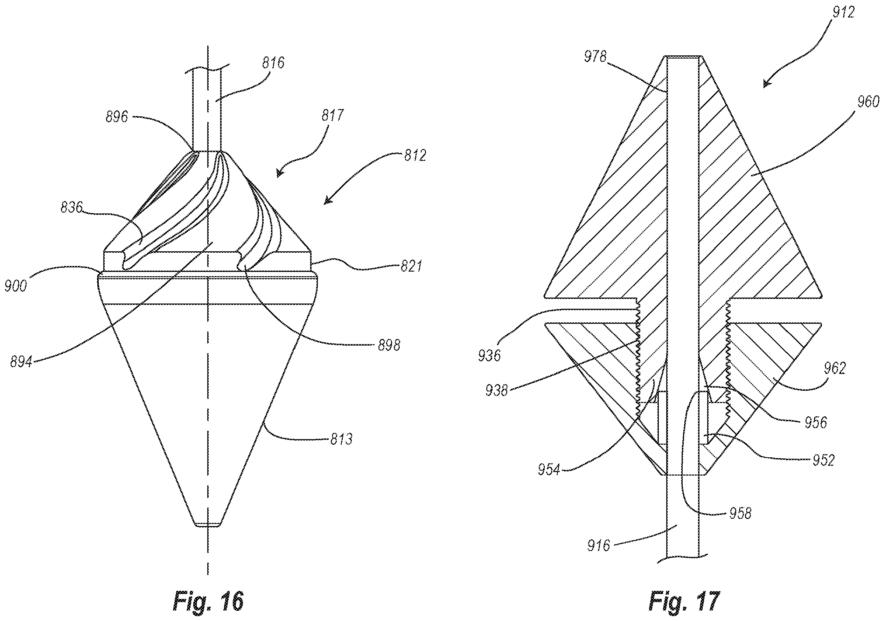

Turning to FIG. 16, there is illustrated an example of a distal cap 812 in accordance with the invention. The discussion related to the other distal caps, guidewire receiving members, etc., described herein are also applicable to the discussion of this example of the distal cap 812, and vice versa. For instance, and not by way of limitation, the distal cap 812 is generally atraumatic and can have at least a portion, in one configuration, that is formed or fabricated of a polymeric material that will limit damage to bodily tissue.

During some medical procedures there is a possiblity that the distal cap of the intravascular delivery device system might being entangled with bodily structures. For instance, a proximal and distal movement of the distal tip might not be sufficient to disentangle the distal tip from the chordae/papillary muscle during a cardiac procedure.

To aid with the releasing of the distal tip of the intravascular delivery device system, the distal cap 812 includes proximal tapered end 817 with a thread 836 extending over substantially all of the proximal tapered end 817 and optionally to and on the shoulder 821 that stops the distal cap 812 entering too far into the outer sleeve 114 (FIG. 1). A crest 894 of the thread 836 increases in cross section from a proximal end 896 of the distal cap 812 to the shoulder 821, which has the form of a wall extending proximally from the tapered distal end 813, as the crest 894 spirals about the longitudinal axis of the distal cap 812. The root 898 of the distal cap 812 also spirals about the longitudinal axis. An atraumatic portion 900 transitions the tapered distal end 813 to the shoulder 821 and provides a curved surface that limits damage to the bodily tissue into which the distsal cap 812 becomes entangled.

The thread 836, in one configuration, is smooth with a thread angle of between 30° - 60° and a pitch of about 15mm to about 30mm. With this configruation, the distal cap 812 attached to a guidewire receiving member 816 can be "screwed out" of engagement with the bodily tissue by turning the guide wire lumen at its proximal end. This rotational motion disentangles the distal cap 812 from the chordae/papillary muscles, for instance.

As mentioned above, the distal cap 812 is attached to the guidewire receiving member 816. For instance, the distal cap 812 can be attached through welding, laser welding, adhesive bonding, engagement of complementary structures (such as, in one example, outer surface threads formed on the guidewire receiving member 816 and complementary threads formed on an inner surface of a channel of the distal cap 812), and combinations of the same. In one configuration, the distal cap 812 is threadably attached to a complementary thread on an outer or inner surface of the guidewire receiving member 816, similar to the attachment described in relation to the other distal caps described in relation to FIGS. 7-8B.

Another manner of connecting the distal cap to the guidewire receiving member is illustrated in FIG. 17. The discussions related to the other distal caps, guidewire receiving members, etc., described herein are also applicable to the discussion of this example of the distal cap 912, and vice versa. For instance, and not by way of limitation, the distal cap 912 is generally atraumatic and can have at least a portion, in one configuration, that is formed or fabricated of a polymeric material that will limit damage to bodily tissue.

As shown, the distal cap 912 is mounted to a guidewire receiving member 916 using an intermediate locking ring 952. The locking ring 952 can be slid onto the guidewire receiving member 916 to be disposed between a distal cap end portion 960 and a distal cap proximal portion 962, with the guidewire receiving member 916 disposed in a channel 978 formed by the distal cap end portion 960 and a distal cap proximal portion 962. Following sliding or positioning of the distal cap proximal portion 962, the locking ring 952 can be slid or positioned beside the distal cap proximal portion 962 on the guidewire receiving member 916. As a threaded portion 936 of the distal cap end portion 960 threadably engages with the threaded portion 938 of the distal cap proximal portion 962 when the distal cap end portion 960 is advanced along the guidewire receiving member 916, the locking ring 952 is forced into contact with the outer surface of the guidewire receiving member 916. In particular, a proximal end 954 of the distal cap end portion 960 includes a tapered recess 956 that receives a distal end of the locking ring 952 as the distal cap end portion 960 and the distal cap proximal portion 962 are brought into engagement. This distal end has a sharpened or cutting edge 958 that is embedded into or carves into the outer surface of the guidewire receiving member 916 to prevent relative movement between the distal cap 912 and the guidewire receiving member 916. In this way, the softer or more atraumatic distal cap end portion 960 is screwed into a more rigid distal cap proximal portion 962, with the locking ring 952, made from a metallic or polymeric material, deforming to secure the distal cap 912 to the guidewire receiving member 916.

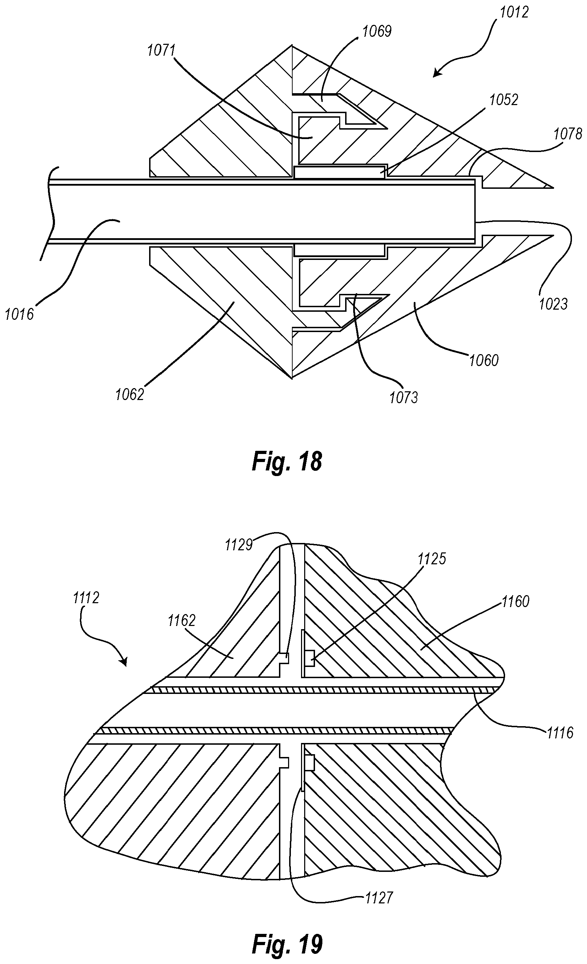

In another configuration, as illustrated in FIG. 18, the distal cap can have a two-part construction with a distal cap end portion in "snap-fit" engagement with a distal cap proximal portion, similar to that described and illustrated in FIGS. 11-12. The discussions related to the other distal caps, guidewire receiving members, etc., described herein are also applicable to the discussion of this example of the distal cap 1012, and vice versa. For instance, and not by way of limitation, the distal cap 1012 is generally atraumatic and can have at least a portion, in one configuration, that is formed or fabricated of a polymeric material that will limit damage to bodily tissue.

As shown in FIG. 18, the distal cap 1012 is mounted to a guidewire receiving member 1016 using a locking ring 1052 mounted to the outer surface of the guidewire receiving member 1016. For instance, the locking ring 1052 is attached through welding, laser welding, adhesive bonding, engagement of complementary structures, and combinations of the same. This locking ring 1052, which can optionally be a radiopaque marker to aid with positioning the distal cap 1012 during a procedure, can function as a stop to prevent excessive advancement of the distal cap end portion 1060 towards the distal cap proximal portion 1062. The locking ring 1052 also provides mechanical interference with the distal cap end portion 1060 to aid with attaching the distal cap 1012 to the guidewire receiving member 1016.

As shown in FIG. 18, a channel 1078 has a stepped form to accommodate the locking ring 1052 and function as a stop for a distal end 1023 of the guidewire receiving member 1016. While two steps are illustrated additional or fewer steps could be included in the channel 1078. In addition, the channel 1078 could optionally include threads that could engage with threads formed on an outer surface of the guidewire receiving member 1016.

With continued reference to FIG. 18, a snap-fit or friction fit locking connection or mechanical connection mounts or attaches the distal cap end portion 1060 to the distal cap proximal portion 1062. One of the distal cap end portion 1060 and the distal cap proximal portion 1062 includes a protruding portion 1069 that deflects upon contact with a mating portion 1071 in the other of the distal cap end portion 1060 and the distal cap proximal portion 1062. Once the protruding portion 1069 passes an end of the mating portion 1071, the temporary deflection ceases and the protruding portion 1069 returns toward the pre-deflection position and the end of the protruding portion 1069 is received into a depression 1073 or undercut, thereby locking the distal cap end portion 1060 to the distal cap proximal portion 1062. Various snap fit or friction fit joints can be used to attach the distal cap end portion 1060 to the distal cap proximal portion 1062.

To provide added security to the junction between the distal cap end portion and the distal cap proximal portion of the two-part distal caps described herein, and those that could be identified based upon the general teaching of this disclosure, it is further possible to glue or bond the distal cap to the guidewire receiving member. The discussions related to the other distal caps, guidewire receiving members, etc., described herein are also applicable to the discussion of this example of the distal cap 1112, and vice versa. For instance, and not by way of limitation, the distal cap 1112 is generally atraumatic and can have at least a portion, in one configuration, that is formed or fabricated of a polymeric material that will limit damage to bodily tissue.

As shown in FIG. 19, the distal cap end portion 1160 includes a cavity or recess 1125 filled with a sticking or fixing liquid or material, such as an adhesive or glue, such as cyanoacrylate or other suitable glues, and which is covered by a film 1127. The film 1127 is punctured by a protrusion 1129 extending from a surface of the distal cap proximal portion 1162 when the distal cap end portion 1160 and the distal cap proximal portion 1162 are snapped or screwed together. When the film 1127 is punctured, the sticking or fixing liquid or material flows from the cavity 1125 into the space between the distal cap end portion 1160, the distal cap proximal portion 1162, and/or the guidewire receiving member 1116 to join them together.

An intravascular device delivery system according to the present disclosure may allow delivery of larger intravascular devices and/or through smaller bodily conduits. The intravascular device delivery system may allow for new and/or improved procedures with less risk to the patient and greater ease of operation to the medical professional.

The articles "a," "an," and "the" are intended to mean that there are one or more of the elements in the preceding descriptions. The terms "comprising," "including," and "having" are intended to be inclusive and mean that there may be additional elements other than the listed elements. Additionally, it should be understood that references to "one arrangement" or "an arrangement" are not intended to be interpreted as excluding the existence of additional arrangements that also incorporate the recited features. Numbers, percentages, ratios, or other values stated herein are intended to include that value, and also other values that are "about" or "approximately" the stated value, as would be appreciated by one of ordinary skill in the art encompassed by the present disclosure. A stated value should therefore be interpreted broadly enough to encompass values that are at least close enough to the stated value to perform a desired function or achieve a desired result. The stated values include at least the variation to be expected in a suitable manufacturing or production process, and may include values that are within 5%, within 1%, within 0.1%, or within 0.01% of a stated value.

The described arrangements are to be considered as illustrative and not restrictive. The scope of the disclosure is, therefore, indicated by the appended claims rather than by the foregoing description.

## Claims

1. An intravascular device delivery system (100), the system (100) comprising:
an elongated member (102) with a proximal end (104), a distal end (106), and a longitudinal axis (126) therebetween, the elongated member (102) including:
an outer sleeve (114) extending from the proximal end (104) to the distal end (106),
a guidewire receiving member (116, 816) extending from the proximal end (104) to the distal end (106), and
a distal cap (112, 812) longitudinally fixed to the guidewire receiving member (116, 816) and configured to engage with a distal end of the outer sleeve (114), the distal cap (112, 812) having a tapered distal end (113, 813) that tapers in the distal direction to form a tapered, atraumatic distal tip, a proximal tapered end (117, 817) that tapers in the proximal direction, and a shoulder (121, 821) positioned between the distal tapered end (113, 813) and the proximal tapered end (117, 817), **characterised in that** the proximal tapered end (117, 817) comprises a thread (836), the thread (836) having a crest (894) that increases in cross section from a proximal end (896) of the distal cap (112, 812) to the shoulder (121, 821) and that spirals about a longitudinal axis of the distal cap.

2. The system (100) of claim 1, further comprising a handle (108) connected to a proximal end of the guidewire receiving member (116).

3. The system (100) of claim 2, wherein the handle (108) includes a proximal end plate (132) that is longitudinally moveable relative to a body (128) of the handle (108).

4. The system (100) of claim 3, wherein the proximal end plate (132) is selectively fixable longitudinally relative to the body (128) of the handle (108).

5. The system (100) of claim 4, wherein the guidewire receiving member (116, 816) is selectively fixed to the proximal end plate (132).

6. The system (100) of claim 1, wherein the guidewire receiving member (116, 816) is a cable tube.

7. The system (100) of claim 1, further comprising a guidewire (240) having one or more torque transmission features (242) protruding in a transverse direction from a longitudinal axis of the guidewire (240); and, optionally, wherein the distal cap comprises complementary receiving recesses (244).

8. The system (100) of claim 1, wherein the distal cap (112, 812) and the outer sleeve (114) comprise complementary mechanical connection features.

9. The system (100) of claim 1, wherein the distal cap comprises a distal cap end portion (960) and a distal cap proximal portion (962), the distal cap end portion (960) mechanically engaging with a locking ring (952) selectively mounted to the guidewire receiving member.

10. The system (100) of claim 9, wherein the locking ring (952) slidable cooperates with the guidewire receiving member.

11. The system (100) of claim 9, further comprises complementary recess (1125) in one of the distal cap end portion and the distal cap proximal portion and a protrusion (1129) in the other of the distal cap end portion and the distal cap proximal portion; and, optionally, further comprising a fixing material to attach the distal cap end portion and the distal cap proximal portion to the guidewire receiving member.

## Patentansprüche

1. Zuführsystem für intravaskuläre Vorrichtung (100), das System (100) umfassend:
ein längliches Element (102) mit einem proximalen Ende (104), einem distalen Ende (106) und einer Längsachse (126) dazwischen, wobei das längliche Element (102) enthält:
eine äußere Hülle (114), die sich von dem proximalen Ende (104) zum distalen Ende (106) erstreckt,
ein Führungsdrahtaufnahmeelement (116, 816), das sich vom proximalen Ende (104) zum distalen Ende (106) erstreckt, und
eine distale Kappe (112, 812), die längs am Führungsdrahtaufnahmeelement (116, 816) befestigt ist und ausgestaltet ist, ein distales Ende der äußeren Hülle (114) in Eingriff zu nehmen, wobei die distale Kappe (112, 812) ein verjüngtes distales Ende (113, 813), das sich in distaler Richtung zur Bildung einer verjüngten, atraumatischen distalen Spitze verjüngt, ein proximales verjüngtes Ende (117, 817), das sich in proximaler Richtung verjüngt, und eine Flanke (121, 821) aufweist, die zwischen dem distalen verjüngten Ende (113, 813) und dem proximalen verjüngten Ende (117, 817) positioniert ist, **dadurch gekennzeichnet, dass**
das proximale verjüngte Ende (117, 817) ein Gewinde (836) umfasst,
wobei das Gewinde (836) einen Kamm (894) aufweist, der seinen Querschnitt von einem proximalen Ende (896) der distalen Kappe (112, 812) zur Flanke (121, 821) vergrößert und der sich um eine Längsachse der distalen Kappe schraubt.

2. System (100) nach Anspruch 1, ferner umfassend einen Griff (108), der mit einem proximalen Ende des Führungsdrahtaufnahmeelements (116) verbunden ist.

3. System (100) nach Anspruch 2, wobei der Griff (108) eine proximale Endplatte (132) enthält, die relativ zu einem Körper (128) des Griffs (108) längs beweglich ist.

4. System (100) nach Anspruch 3, wobei die proximale Endplatte (132) relativ zu dem Körper (128) des Griffs (108) selektiv längs befestigbar ist.

5. System (100) nach Anspruch 4, wobei das Führungsdrahtaufnahmeelement (116, 816) an der proximalen Endplatte (132) selektiv befestigt ist.

6. System (100) nach Anspruch 1, wobei das Führungsdrahtaufnahmeelement (116, 816) ein Kabelrohr ist.

7. System (100) nach Anspruch 1, ferner umfassend einen Führungsdraht (240), der ein oder mehrere Drehmomentübertragungsmerkmale (242) aufweist, die in querlaufender Richtung von einer Längsachse des Führungsdrahts (240) vorspringen; und, optional, wobei die distale Kappe komplementäre Aufnahmeausnehmungen (244) umfasst.

8. System (100) nach Anspruch 1, wobei die distale Kappe (112, 812) und die äußere Hülle (114) komplementäre mechanische Verbindungsmerkmale umfassen.

9. System (100) nach Anspruch 1, wobei die distale Kappe einen Endabschnitt (960) der distalen Kappe und einen proximalen Abschnitt (962) der distalen Kappe umfasst, wobei der Endabschnitt (960) der distalen Kappe einen Sicherungsring (952), der am Führungsdrahtaufnahmeelement selektiv montiert ist, mechanisch in Eingriff nimmt.

10. System (100) nach Anspruch 9, wobei der Sicherungsring (952) mit dem Führungsdrahtaufnahmeelement verschiebbar zusammenwirkt.

11. System (100) nach Anspruch 9, ferner umfassend eine komplementäre Ausnehmung (1125) in entweder dem Endabschnitt der distalen Kappe oder dem proximalen Abschnitt der distalen Kappe und einen Vorsprung (1129) in dem jeweils anderen des Endabschnitts der distalen Kappe oder des proximalen Abschnitts der distalen Kappe; und, optional, ferner umfassend ein Befestigungsmaterial zur Befestigung des Endabschnitts der distalen Kappe und des proximalen Abschnitts der distalen Kappe am Führungsdrahtaufnahmeelement.

## Revendications

1. Système de mise en place de dispositif intravasculaire (100), le système (100) comprenant :
un élément allongé (102) avec une extrémité proximale (104), une extrémité distale (106) et un axe longitudinal (126) entre celles-ci, l'élément allongé (102) incluant :
un manchon externe (114) s'étendant de l'extrémité proximale (104) à l'extrémité distale (106),
un élément de réception de fil-guide (116, 816) s'étendant de l'extrémité proximale (104) à l'extrémité distale (106), et
un capuchon distal (112, 812) fixé longitudinalement à l'élément de réception de fil-guide (116, 816) et configuré pour venir en prise avec une extrémité distale du manchon externe (114), le capuchon distal (112, 812) présentant une extrémité distale conique (113, 813) qui est effilée dans la direction distale pour former une pointe distale effilé atraumatique, une extrémité effilée proximale (117, 817) qui est effilée dans la direction proximale, et un épaulement (121, 821) positionné entre l'extrémité effilée distale (113, 813) et l'extrémité effilée proximale (117, 817), **caractérisé en ce que**
l'extrémité effilée proximale (117, 817) comprend un filetage (836),
le filetage (836) présentant une crête (894) dont la section transversale augmente depuis une extrémité proximale (896) du capuchon distal (112, 812) jusqu'à l'épaulement (121, 821) et qui est en spirale autour d'un axe longitudinal du capuchon distal.

2. Système (100) selon la revendication 1, comprenant en outre un poignée (108) reliée à une extrémité proximale de l'élément de réception de fil-guide (116).

3. Système (100) selon la revendication 2, dans lequel la poignée (108) inclut une plaque d'extrémité proximale (132) qui est mobile longitudinalement par rapport à un corps (128) de la poignée (108).

4. Système (100) selon la revendication 3, dans lequel la plaque d'extrémité proximale (132) peut être fixée de manière sélective longitudinalement par rapport au corps (128) de la poignée (108).

5. Système (100) selon la revendication 4, dans lequel l'élément de réception de fil-guide (116, 816) est fixé de manière sélective à la plaque d'extrémité proximale (132).

6. Système (100) selon la revendication 1, dans lequel l'élément de réception de fil-guide (116, 116) est un tube de câble.

7. Système (100) selon la revendication 1, comprenant en outre un fil-guide (240) présentant une ou plusieurs caractéristiques de transmission de couple (242) faisant saillie dans une direction transversale à partir d'un axe longitudinal du fil-guide (240) ; et facultativement dans lequel le capuchon distal comprend des évidements de réception complémentaires (244).

8. Système (100) selon la revendication 1, dans lequel le capuchon distal (112, 812) et le manchon externe (114) comprennent des caractéristiques de liaison mécanique complémentaires.

9. Système (100) selon la revendication 1, dans lequel le capuchon distal comprend une partie d'extrémité de capuchon distal (960) et une partie proximale de capuchon distal (962), la partie d'extrémité de capuchon distal (960) venant en prise mécaniquement avec une bague de verrouillage (952) montée sélectivement sur l'élément de réception de fil-guide.

10. Système (100) selon la revendication 9, dans lequel la bague de verrouillage (952) coopère de manière coulissante avec l'élément de réception de fil-guide.

11. Système (100) selon la revendication 9, comprenant en outre un évidement complémentaire (1125) dans l'une de la partie d'extrémité de capuchon distal et de la partie proximale de capuchon distal et une saillie (1129) dans l'autre de la partie d'extrémité de capuchon distal et de la partie proximale de capuchon distal ; et facultativement comprenant en outre un matériau de fixation pour fixer la partie d'extrémité de capuchon distal et la partie proximale de capuchon distal à l'élément de réception de fil-guide.
